(19) Europäisches Patentamt
European Patent Office
Office européen des brevets

(11) Publication number: **0 500 802 B1**

(12) # EUROPEAN PATENT SPECIFICATION

(45) Date of publication of patent specification: **24.05.95**

(51) Int. Cl.[6]: **A61K 9/127**, A61K 31/35

(21) Application number: **91901087.6**

(22) Date of filing: **30.10.90**

(86) International application number:
**PCT/US90/06291**

(87) International publication number:
**WO 91/06310 (16.05.91 91/11)**

The file contains technical information submitted after the application was filed and not included in this specification

(54) **LIPOSOMAL COMPOSITIONS.**

(30) Priority: **30.10.89 US 429278**

(43) Date of publication of application:
**02.09.92 Bulletin 92/36**

(45) Publication of the grant of the patent:
**24.05.95 Bulletin 95/21**

(84) Designated Contracting States:
**AT BE CH DE DK ES FR GB GR IT LI LU NL SE**

(56) References cited:
**EP-A- 0 084 898**
**US-A- 4 721 612**
**US-A- 4 849 227**
**US-A- 4 917 897**

(73) Proprietor: **THE LIPOSOME COMPANY, INC.**
**One Research Way**
**Princeton Forrestal Center**
**Princeton, NJ 08540 (US)**

(72) Inventor: **BOLCSAK, Lois, E.**
**11 Tower Place**
**Lawrenceville, NJ 08648 (US)**

(74) Representative: **Ahner, Francis et al**
**CABINET REGIMBEAU**
**26, avenue Kléber**
**F-75116 Paris (FR)**

EP 0 500 802 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

STN FILE SERVER, FILE MEDLINE, AN 88252103; A.S. JANOFF et al.:"Characterization of cholesterol hemisuccinate and alpha-tocopherol hemisuccinate vesicles" & Biochim. Biophys. Acta (22nd June 1988), 941(2), 165-75

STN FILE SERVER, FILE CA, & CHEMICAL ABSTRACTS, vol.104, no. 26, abstract no. 230318e, Columbus, Ohio, US; F.C. SZOKA, Jr.: "pHtriggered liposome destabilization utilizing the lamellar-hexagonal phase transition", & POLYM. PREPR. (AM. CHEM. SOC., DIV. POLYM. CHEM.),1986, 27(1), 36-7

STN FILE SERVER, FILE MEDLINE, AN 06547732, 88192732; E.C. SOEHNGEN et al.:"Encapsulation of indomethacin in liposomes provides protection against both gastric and intestinal ulceration when orally administered to rats",& ARTHRITIS RHEUM. MARCH 1988, 31(3), P414-22

US-A4917897

Remington's Pharmaceutical Science, 1975, page 1101

## Description

Background of the Invention

The present invention is directed to liposomal cromolyn formulations comprising cromolyn and neutral pH cholesterol hydrogen succinate lipids. This invention specifically discloses formulations wherein the lipid portion of the liposome comprises lipids such as neutralized cholesterol hydrogen succinate known as cholesterol hemisuccinate (CHS), saturated phospholipids such as hydrogenated soy phosphatidylcholine (HSPC), dipalmitoylphosphatidylcholine (DPPC) or lipid mixtures such as a HSPC-cholesterol, DPPC-cholesterol or dimyristoylphosphatidylcholine (DMPC)-cholesterol.

The term cromolyn as used herein includes all structural analogs and functional derivatives of cromolyn as disclosed in U.S. Patent No. 3,419,578. Functional derivatives of cromolyn include its salts, esters and amides, in particular the disodium salt of cromolyn e.g. disodium cromoglycate or cromolyn sodium.

Cromolyn, whose chemical names include 5,5' -[(2-Hydroxy-1,3-propanediyl)bis-(oxy)]bis[4-oxo-4H-1-benzopyran-2-carboxylic acid]; 5,5'
-[(2-hydroxytrimethylene)dioxy]bis(4-oxo-4H-1-benzopyran-2-carboxylic acid); 5,5'
-(2-hydroxytrimethylenedioxy)bis(4-oxochromene-2-carboxylic acid); 1,3-bis(2-carboxychromon-5-yloxy)-2-hydroxypropane;
1,3-di(2-carboxy-oxochromen-5-yloxy)propan-2-ol; and cromoglycic acid, has the following chemical structure:

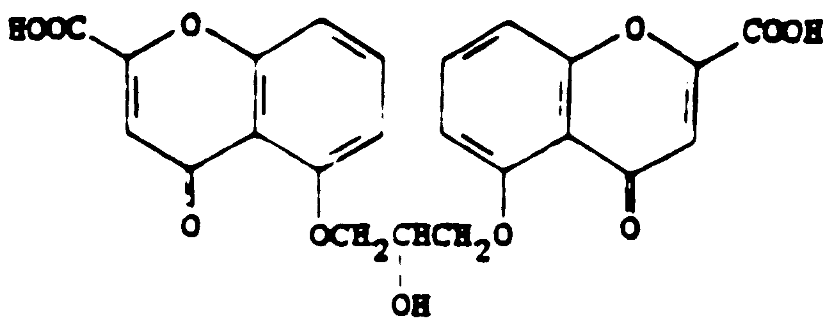

Its method of preparation is described in U.S. Patent No. 3,419,578.

Cromolyn is knob to be useful for the treatment of allergic conditions, e.g. asthma, hay fever and conjunctivitis of allergic origin, e.g. vernal kerato conjunctivitis and allergic rhinitis. Other disclosed uses of disodium cromoglycate include the treatment of viral infections, the treatment of gastro-intestinal diseases e.g. ulcerative colitis and food allergies and the treatment of skin conditions, e.g. chronic dermatoses such as eczemas, psoriasis, dermatitis and chronic skin ulcers.

Liposomes are completely closed lipid bilayer membranes containing an entrapped aqueous volume. Liposomes may be unilamellar vesicles (possessing a single bilayer membrane) or multilamellar vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol. Biol., 1965, 12:238-252) involves suspending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles.

Unilamellar vesicles may be produced using an extrusion apparatus by a method described in Cullis et al., PCT Application No. WO87/00238, published January 16, 1986, entitled "Extrusion Technique for Producing Unilamellar Vesicles" incorporated herein by reference. Vesicles made by this technique, called LUVETS, are extruded under pressure through a membrane filter. Vesicles may also be made by an extrusion technique through a 200 nm filter. Such vesicles are known as $VET_{200}S$.

Another class of liposomes that may be used in the present invention are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4,588,579 to Fountain, et al. and frozen and thawed multilamellar vesicles (FATMLV) wherein the vesicles are exposed to at least one freeze and thaw cycle; this procedure is described in Bally et al., PCT Publication No. WO87/00043, January 15, 1987, entitled "Multilamellar Liposomes Having Improved Trapping Efficiencies".

Cromolyn (e.g., sodium cromoglycate) liposomal formulations are known in the art. European Patent Publication No. 0 084 898 published August 3, 1983, discloses pharmaceutical compositions comprising liposomes and sodium cromoglycate for treating asthma, hay fever and vernal kerato conjunctivitis. However, liposomal cromolyn compositions having the formulations of the instant invention such as neutral pH CHS lipids or DMPC-Chol or HSPC-Chol lipid mixtures are new.

## Summary of the Invention

The present invention is defined by the claims. The lipid component of the inventive compositions comprising pH neutral cholesterol hydrogen succinate (CHS); and in addition to that may comprise saturated phospholipids such as for example hydrogenated soy phosphatidylcholine (HSPC), dipalmitoyl phosphatidylcholine (DPPC), hydrogenated soy phosphatidylcholine (HSPC); dimyristoylphosphatidylcholine (DMPC), or a hydrogenated soy phosphatidylcholine and cholesterol mixture (HSPC/Chol); or a dimyristoylphosphatidylcholine and cholesterol mixture (DMPC/Chol) or a dipalmitoyl phosphatidylcholine and cholesterol mixture (DPPC/Chol).

The inventive liposomal compositions may be incorporated into a pharmaceutical composition by combining or admixing them with a pharmaceutically carrier or diluent.

The liposomal cromolyn compositions of the present invention are especially useful for treating allergic conjunctivitis or allergic rhinitis.

A preferred CHS cromolyn liposome formulation additionally contains disodium EDTA and phosphate buffer to a pH around about pH 7.0. It may also include benzalkonium chloride and phenylethyl alcohol.

A preferred cromolyn liposome composition containing a saturated phospholipid and cholesterol also contains phosphate buffer to a pH around about neutrality, e.g., pH 7.0. Such composition may also contain disodium EDTA. The phosphate buffer is preferably sodium phosphate monobasic and sodium phosphate dibasic. The ratio of cromolyn to lipid in such compositions ranges from about 0.005 to about 20.0, more preferably from about 0.071 to about 0.667, and most preferably is about 0.2.

The saturated lipid of the invention is dimyristoylphosphatidylcholine (DMPC), dipalmitoylphosphatidylcholine (DPPC), or hydrogenated soy phosphatidylcholine (HSPC).

The liposome composition may contain a preservative, which can be phenylmercuric acetate, benzalkonium chloride, or chlorbutanol. It may also be phenylmercuric acetate. The preservatives may be employed alone or in combination, and the preparations may also contain methylparaben and propylparaben.

When the saturated lipid is dimyristoylphosphatidylcholine (DMPC), the formulation may also contain phenylmercuric acetate, and benzalkonium chloride or phenylethyl alcohol. Alternatively, the formulation of DMPC/cholesterol may contain benzalkonium chloride and thimersol.

Pharmaceutical composition comprising the liposome composition of any of the above formulations are also contamplated, wherein the composition comprises a pharmaceutically acceptable carrier or diluent.

## Brief Description of the Drawing

Figure 1 is a schematic for the preparation of cromolyn liposomes by aseptic process.

Figure 2 is a graphic presentation of the tissue concentration (mg Cromolyn/g or ml tissue) of the Cromolyn Free Drug over time (also found in Table 1).

## Detailed Description of the Invention

The lipids which can be used in the liposome formulations of the present invention are the unsaturated and saturated phospholipids such as phosphatidylcholine (PC), hydrogenated soy phosphatidylcholine (HSPC), phosphatidylethanolamine (PE), phosphatidylserine (PS), phosphatidylglycerol (PG), phosphatidic acid (PA), phosphatidylinositol (PI), sphingomyelin (SPM), and the like, alone or in combination. Preferred phospholipids include dimyristoylphosphatidylcholine (DMPC), dipalmitoyl phosphatidylcholine (DPPC), and

hydrogenated soy phosphatidylcholine (HSPC). These lipids can be used alone or in combination with cholesterol. The phospholipids can be synthetic or derived from natural sources such as egg or soy.

The liposomes can also contain other steroid components such as derivatives of cholesterol, coprostanol, cholestanol, or cholestane, and combinations of PC and cholesterol. They contain neutral-pH cholesterol hemisuccinate. CHS containing liposomes and their salt forms may generally be prepared by any method known in the art for preparing liposomes containing these sterols. In particular, see the procedures of Janoff, et al., U.S. Pat. No. 4,721,612 issued January 26, 1988, entitled "Steroidal Liposomes".

Derivatized sterols of the invention have a general structure wherein a core molecule (A) comprises a sterol, a steroid, a bile acid or an intermediate of sterol metabolism; a chemical bridge (B) such as an ester, ether, sulfide (thioether), alkyl (methylene, ethylene, propylene, butylene, etc.), amide, amine, phosphonate, thioester, sulfonate and disulfide between the core molecule (A) and an anchor molecule (C); C is selected from among linear branched or cyclic structures terminating in a charged or hydrophilic group (D); (D) the proximal anchor, is a hydrophilic or charged group, preferably a charged group, selected from among a carboxylate, carbonate, phosphate, sulfonate, sulfate, borate, protonated amine, quarternary amine, imidazole, sulfonium, pyrrolidone, pyrrole and pyridinium; and a counter ion (E) to said group (D) when (D) is charged, selected from among an acetate, sulfate, sulfonate, phosphate, arsenate, borate, nitrate, chlorate, a metal ion, charged amine, including a quarternary amine, imidazole, sulfonium and diazonium salt, among others, including larger molecules, for example, amino acids, EDTA, TRIS, choline, peptides, nucleosides, nucleotides and oligo and polynucleotides, among others.

The liposomes can further comprise any of the abovenamed lipids alone or in combination. A preferable lipid combination for the liposome compositions of the invention is DMPC/cholesterol or HSPC/cholesterol, in a 16:1 to about a 1:1 weight ratio, more preferably about a 10.0 : 2.4 %w/v ratio.

The cromolyn to lipid weight ratios of the liposomal compositions of the present invention range about .005 (0.1 %w/v drug, 20.0 %w/v lipid) to about 20.0 (10 %w/v drug, 0.5 %w/v lipid), more preferably from about .071 (1.0 %w/v drug, 14.0 %w/v lipid) to about .667 (4.0 %w/v drug, 6.0 %w/v lipid), most preferably about .2 (2.0 %w/v drug, 10.0 %w/v lipid).

The liposome-cromolyn formulations can additionally contain one or more antimicrobial preservative solutions, such as sorbic acid, p-hydroxybenzoic acid methyl, ethyl, butyl or propyl esters, benzyl alcohol, chlorbutanol, benzalkonium chloride (BAK), phenethyl alcohol, and the mercurial preservatives such as phenylmercuric acetate. Other preservatives include boric acid, benzethonium chloride, butylparaben, cetylpyridinium chloride, chlorhexidine digluconate, methylparaben, propylparaben and thimersol. The preservative is preferably present in the final preparation at about 0.01-0.1% (w/v), most preferably 0.05%. The amount of preservative needed can be determined by its performance in the USP Antimicrobial Effectiveness test (AME), The United States Pharmacopeia The National Formulary, The United States Pharmacopeial Convention, Inc., Rockville, MD, which is incorporated herein by reference, and by its structural and chemical compatibility with other components in the preparation.

The preservative solution may also comprise enhancer substances such as ethylenediamine tetraacetic acid (EDTA) at about 0.01-0.1% (w/v), preferably 0.05% (w/v). Preferably in the present invention, sorbic acid: EDTA (1 : 1) is used.

The liposome-cromolyn formulations of the invention can additionally contain an antioxidant, such as for example butylated hydroxytoluene (BHT).

Any aqueous solutions such as for example aqueous buffers can be used in the practice of the instant invention. Especially preferable are those pharmaceutically acceptable solutions such as physiological saline, citrate, carbonate, phosphate or tris(hydroxymethyl)aminomethane (Tris). The concentrations of these solutions will depend upon other components of the formulation and their concentrations, which solutions will result in a tonicity as close to physiological as possible; such being within the knowledge of one having ordinary skill in the formulations art.

The solutions preferably employed in the present invention are phosphate or citrate. Where buffer capacity near about pH 7.0 is required, phosphate buffer was employed. The preferred phosphate buffers are sodium phosphate, monobasic and sodium phosphate, dibasic. Most preferably these buffers are employed together in amounts which will adjust the final pH of the liposomal cromolyn compositions to about pH 7.0. Citrate was selected for buffering at pH 5.5. These two pH values fall into the preferred range for cromolyn. Sodium chloride can be used to adjust tonicity.

Compounds that are hydrophilic polymer viscosity enhancers such as polyvinyl alcohol, polyvinyl pyrolidone or hydroxypropyl methyl cellulose, which also enhances the retention of liposomes on the tissues can be used. These compounds are used in about a 0.05-1.5% (w/v) concentration of final preparation.

The cromolyn liposomes of the invention can be size reduced or homogenized by a variety of methods. A liposome solution is passed under pressure a multiple of times (preferably about 10 times) through a stainless steel filter having a nominal pore size of about 500 nm. Alternatively, the liposomes can be extruded by passage through a uniform-pore size filter according to the LUVET method, described hereinabove, disclosed in Cullis et al., PCT Publication No. 87/00238, January 16, 1986. Alternatively, the liposomes of the invention can be homogenized using an apparatus such as the Mantin-Gaulin homogenizer or the Silverson homogenizer.

The liposomes of the present invention may be dehydrated thereby enabling storage for extended periods of time until use. Standard freeze-drying equipment or equivalent apparatus may be used to lyophilize the liposomes. Liposomes may also be dehydrated simply by placing them under reduced pressure and allowing the suspending solution to evaporate. Alternatively, the liposomes and their surrounding medium may be frozen in liquid nitrogen prior to dehydration. Such dehydration may be performed in the presence of one or more protectants such as protective sugars, according to the process of Janoff et al., U.S. Patent No. 4,880,635, issued November 14, 1989, and PCT publication No. WO86/01103, published February 27, 1986.

During preparation of the liposomes, organic solvents may be used to solubilize the lipids. Suitable organic solvents are those with a variety of polarities and dielectric properties, which solubilize the lipids, and include but are not limited to halogenated, aliphatic, cycloaliphatic, or aromatic-aliphatic hydrocarbons, such as benzene, chloroform, methylene chloride, or alcohols, such as methanol, ethanol, and solvent mixtures such as benzene:methanol (70:30 v/v). As a result, solutions (mixtures in which the lipids and other components are uniformly distributed throughout) containing the lipids are formed. Solvents are generally chosen on the basis of their biocompatability, low toxicity, and solubilization abilities. For the purposes of the instant invention, the organic solvent methylene chloride is particularly preferred.

Examples 2-4 hereinbelow disclose methods for forming the liposomal cromolyn formulations of the invention. For an aseptic process, a schematic diagram of one method of preparation of the liposomal cromolyn of the invention is presented in Figure 1.

In one method of making the liposome-cromolyn compositions of the present invention, lipids are dissolved in organic solvent while cromolyn (sodium cromoglycate) is dissolved in aqueous solution such as for example water. The organic and aqueous phases are transferred to a round bottom flask and the solvent removed by vacuum evaporation. The resulting preparation can then be homogenized using any device suitable for such a process, e.g., a Silverson homogenizer. If viscosity enhancers, antioxidants or antimicrobial agents are employed, they can be admixed with the aqueous solution of cromolyn (sodium cromoglycate) prior to its admixture with the organic phase. Water insoluble components (e.g., such as the antioxidant) can be admixed with the lipid phase in organic solvent).

As examples, formulations of the lipid-cromolyn compositions can comprise for example HSPC : cholesterol in about 10:2.4 to about a 20:4.8 %w/v. DMPC : cholesterol can be used in about a 10:2.4 %w/v ratio. DPPC : cholesterol can be used in about a 10:2.4 %w/v concentration, and EPC can be used either alone or in a 10:30 to 20:60 %w/v concentration with cholesterol. PH-neutral CHS can be used alone in the formation of cromolyn - CHS liposomes of the invention.

As a further example of the methods of the invention, where DMPC-cholesterol is employed as the lipid phase, the lipid used can comprise DMPC and cholesterol in the proportions recited above, admixed in organic solvent. The aqueous phase can comprise 2.0 %w/v sodium cromoglycate. The composition can also contain preservatives such as for example, benzalkonium chloride 0.01 % w/v, and chlorobutanol 0.5 %w/v, and enhancers such as disodium edetate (EDTA) 0.013 %w/v.

HSPC can be present at 10 %w/v, cholesterol at 2.4 %w/v, and the drug present at 2.0 %w/v cromolyn. Preservatives may or may not be present in the compositions. Anti-oxidants can also be present, for example, butylated hydroxytoluene at 0.001 %w/v.

The cromolyn formulation of the invention is neutral pH-CHS which can be present at 10.0 %w/v, with cromolyn at 2.0 %w/v. Preservatives can also be present in these compositions. Anti-oxidants such as butylated hydroxytoluene can also be employed for example at 0.001 %w/v.

Examples of formulations of the invention are shown in appended Tables 3 through 9 and 11 through 20. These liposome formulations are made by the general methods as recited hereinabove and more specifically by the methods disclosed in Examples 2, 3 and 4 or Figure 1 (for an aseptic batch process) of the invention as recited hereinbelow. Formulations of the invention were tested for anitmicrobial effectiveness by the USP National Formulary methods. Tables 11, 12 and 20 also show formulations of the invention that did not pass the USP Antimicrobial Effectiveness Testing (referenced hereinabove), and thus are not preferable for use in the present invention.

A therapeutically effective amount of the compositions herein will be understood to mean a sufficient amount to achieve a physical or physiological response over a period of time. The therapeutically effective amount of a given pharmacological agent will vary with the purpose of the administration, the particularities of the recipient and other factors, especially those related to sustained release, well known in the art.

The liposomes of the present invention can be used therapeutically in mammals, including man, in the treatment of conditions which can be ameliorated by cromolyn in its bioactive form. These conditions include but are not limited to allergic conjunctivitis and allergic rhinitis. In such a use as a pharmaceutical composition, the liposomes of the invention can be admixed with a pharmaceutically acceptable carrier or diluent.

The mode of administration of the preparation may determine the sites and cells in the organism to which the compound will be delivered. Liposomes can be administered alone but will generally be administered in admixture with a pharmaceutical carrier selected with regard to the intended route of administration and standard pharmaceutical practice. The preparations may be injected parenterally, for example, intra-arterially, subcutaneously, intramuscularly or intravenously. The preparations may also be administered via oral, subcutaneous, or intramuscular routes, or by inhalation. For parenteral administration, they can be used, for example, in the form of a sterile aqueous solution which may contain other solutes, such as for example, salts or glucose or any other solutes which are biocompatible. Other uses, depending upon the particular properties of the preparation, may be envisioned by those skilled in the art.

A preferred mode of administration is via eye drops in a form such that the eye drops can be administered using a standard ophthalamic dropper or dropper bottle. Another preferred mode of administration is via nasal spray or a nebuliser.

For administration to humans in the palliative or therapeutic treatment of disease states, the prescribing physician will ultimately determine the appropriate dosage for a given human subject, and this can be expected to vary according to the age, weight, and response of the individual as well as the nature and severity of the patient's disease. The dosage of the drug in liposomal form will generally be about that necessary for the therapeutic treatment of the disease state or condition. In some cases, however, it may be necessary to administer doses outside these limits.

EXAMPLE 1

A. Preparation of test article. The sodium cromoglycate free drug test article used in the ocular biodistribution studies (Examples 1, 2 and 3) was prepared according to the following formula:

| | |
|---|---|
| Sodium cromoglycate | 2.0 %w/v |
| EDTA-disodium | 0.01 %w/v |
| Benzalkonium Chloride | 0.01 %w/v |
| Phenylethyl Alcohol | 0.4 %w/v |
| Sterile Water | qs |
| pH 5.4 | |

B. The [propane-2-$^{14}$C]cromolyn sodium used in the biodistribution studies was prepared as follows: 1,3-dichloro[2-$^{14}$C]propan-2-ol was reacted with 2,6-dihydroxyacetophenone to give 1,3-bis (2-acetyl-3-hydroxy phenoxy)-2-hydroxy[2-$^{14}$C]propane. This was treated with base and diethyl oxalate followed by acid catalysed cyclisation to give diethyl [propane-2-$^{14}$C]cromolyn. Hydrolysis with sodium hydroxide gave the final product which was purified by crystallisation from water acetone.

C. Specification and Handling of Experimental Animals and Tissues.

1. Description of Experimental Animals: Female, New Zealand white rabbits (Hare Marland) weighing 2-3 kg were used in these studies. Upon receipt each animal was assigned an identification number and examined for gross signs of general health. The animals were individually housed in standard rabbit cages and given access to food and water ad libitum. Animals were received approximately one week before initiation of the study.

Prior to the study the animals were randomly assigned to treatment groups using either a random number table or generator. Immediately before treatment the ocular tissues were examined for signs of irritation. If irritation was observed, either another animal was substituted or the condition noted. Food was removed at the time of treatment Water was available ad libitum throughout.

2. Topical Ocular Instillation of Test Article: Animals were treated unilaterally by topical ocular instillation using a positive displacement pipet. The eyelids were gently retracted and the dose

applied onto the cornea taking care to avoid touching the tissue with the pipet tip. The animal was positioned such that the dose flowed into the lower cul-de-sac. After treatment the animals were returned to their cages.

3. Collection of Plasma Sample: Immediately prior to euthanasia, animals were placed in a standard rabbit restrainer. A small amount of xylene was applied to an eartip to enhance blood flow. The lateral ear vein was lanced and approximately 500 ul of blood collected in a Microtainer Brand Tube with EDTA (Becton Dickinson). The tubes were mixed by inversion and centrifuged in a microfuge for 10 minutes. A 200 ul aliquot of plasma was transferred to a scintillation vial for further processing of the sample.

4. Euthanasia: Animals were euthanized by intravenous injection of a lethal dose of T61 Euthanasia Fluid (Hoechst-Roussel) into a lateral ear vein. Death was verified by absence of respiration, heart beat, and ocular reflexes.

5. Dissection of Ocular Tissues.

a. Removal of Aqueous Humor: Aqueous humor was removed using a 1 ml disposable, plastic syringe equipped with a 5/8"-27g needle. The needle was inserted into the anterior chamber at the limbus. The fluid was gently withdrawn and transferred to a preweighed scintillation vial.

b. Removal of Globe, Conjunctiva, and Nictitating Membrane: The eyelids were retracted using 6" curbed hemostats. Incisions were made at the corners of the eye to facilitate dissection of the conjunctiva. The palpebral conjunctiva and nictitating membrane were dissected free of the eyelids, muscle, and connective tissue using Wilmer-Converse conjunctiva scissors. The conjunctiva and nictitating membrane were grasped with straight Kirby tissue forceps to form a sack around the globe. The sack and its contents were removed from the socket as a unit using heavy curved nucleation scissors. Excess blood was removed from the outside of the sac by gentle blotting with gauze pads.

c. Dissection of Conjunctiva, and Nictitating Membrane: The nictitating membrane was grasped with straight Kirby tissue forceps. The conjunctiva was cut with heavy curved eye scissors along one edge of the nictitating membrane. While applying tension at the nictitating membrane, the conjunctiva was dissected away from the globe using heavy curved eye scissors. The nictitating membrane was separated from the conjunctiva by continuing to cut along the remaining edges. The conjunctiva was inspected and any adherent nonconjunctival tissue removed. The samples were then transferred to preweighed scintillation vials.

d. Removal of Cornea and Iris-Ciliary Body: Using clean instruments, the cornea was removed by cutting along the margin using heavy curved eye scissors and light curved iris forceps. The iris-ciliary body was grasped with clean light curved iris forceps and removed from the globe. Adherent lens and vitreous humor were removed. The tissues were transferred to preweighed scintillation vials.

D. Determination of Radioactivity:

1. Sample Preparation: Samples were prepared and the radioactivity determined by liquid scintillation counting in 20 ml scintillation vials of low potassium borosilcate glass capped with urea-formaldehyde caps with Poly-Seal liners (liners compatible with perchloric acid and hydrogen peroxide). Eighteen ml of Ready Safe Scintillation Cocktail for Aqueous Samples (Beckman) (cocktail having both salt and water compatibility which will result in a clear single phase system), were used per sample.

The radioactivity in the aqueous humor samples was determined without oxidation.

All other tissue types were chemically oxidized prior to scintillation counting. Immediately before use, perchloric acid (70%) and hydrogen peroxide (50%) (v/v) were mixed in the ratio of 1:2 by volume. A 0.3 ml aliquot of the mixture was added to a vial which was then tightly capped and incubated at 70 °C for 3-4 hr. Eighteen ml of scintillation cocktail were added when cool.

To determine the loss of radioactivity on oxidation, control tissues were spiked with aliquots of $^{14}C$-sodium cromoglycate of known activity and processed as indicated above.

2. Scintillation Counting: Radioactivity was determined using a Beckman LS5801 Liquid Scintillation Counter equipped with a single label DMP program. Quench correction was performed by external standardization. In order to utilize counting time more efficiently, low sample rejection was used to eliminate samples with insignificant radioactivity levels. The rejection criterion was a count rate less than twice that of background samples. The remaining samples were either counted to 2 sigma error or for 360 min.

3. Calculations and Statistics: Tissue Concentrations: Tissue concentrations were expressed as %dose/g or ml tissue and as ng Cromolyn/g or ml tissue. In the latter case, it was assumed that all radioactivity was associated with the parent compound. The methods of calculation and shown in

Table 2. The calculated weight means and standard deviations are shown in Table 1.

E. Results:

Thirty (30) rabbits were divided into three groups of ten (10) rabbits each. Groups were sacrificed at 2, 4, or 8 hours after instillation of the test article.

Tissue concentrations were expressed as %dose/g or ml tissue and as ng Cromolyn/g or ml tissue. In the latter case, it was assumed that all radioactivity was associated with the parent compounds. Because Cromolyn has been reported to be nonmetabolizable and the radiolabel was of high purity, the assumption is reasonable. A Summary of the tissue concentrations is found in Table 1 and presented graphically in Figure 2.

Examination of Table 1 indicates that no detectable (ND) Cromolyn levels were found in the iris-ciliary body of the treated eye, the plasma, or the aqueous humor of the untreated eye at any time point. Significant concentrations were detected in all other tissues sampled at 2, 4 and 8 hours post-instillation. Although the number of time points examined were minimal, it appears that the sodium cromoglycate concentrations in the conjunctiva and nictitating membrane declined at a relatively slow rate between 4 and 8 hours (see Figure 2).

EXAMPLE 2

Liposomal-sodium cromoglycate employing CHS liposomes was made according to the following method:

The aqueous phase was prepared by dissolving 02.0 g sodium cromoglycate, .05 g sorbic acid, 0.013 g disodium EDTA and 0.71 g dibasic sodium phosphate in qs ad 100 mL of sterile water for injection. The organic phase was prepared by the suspension of 10.0 g of CHS and 0.001 g BHT in 120 mL of methylene chloride in a round bottom flask.

The round bottom flask was transferred to a 40 °C water bath, and the aqueous phase added thereto. While agitating vigorously, 12.3 mEq of a 10 N aqueous solution of sodium hydroxide was added , and the solvent then removed by vacuum evaporation. The volume was adjusted to 100.0 mL with sterile water for injection.

EXAMPLE 6

OCULAR IRRITATION STUDIES

The degree of ocular irritation of liposomal cromolyn formulations was compared to the ocular irritation of a control saline solution. The test articles employed are described in the sub examples A, B and C which follow.

A. Test System - Female, New Zealand white rabbits supplied by Hare-Marland, which had been acclimated for a minimum of one week were used. Animals were housed one per cage and given access to water and food ad libitum throughout the study. Only animals judged to be healthy on the basis of general observation and fluorescein examination of the cornea were used in the study. Animals found suitable were randomly assigned to treatment groups.

Approximately 0.05 mL of the test article was instilled into the cornea of the right eye of each of the four test rabbits six times per day at hourly intervals for four days. The left eye was untreated to serve as a control.

The degree of ocular irritation was scored prior to each dose and at the end of the dosing period on days 1 through 4 and once daily on days 5 through 8. Fluorescein examination was performed after the final dose on day 4.

Each animal was graded for ocular lesions of the cornea, iris, conjunctiva, taking into consideration redness, chemosis and discharge, on the Draize Scale (See Table 10), at the times specified hereinabove.

A

The test article of 0.9% sodium chloride for injection was instilled in the right eye of the 4 test animals as described hereinabove. This test articles was very well tolerated and no treatment related effects were noted.

B

The test article of the DMPC/cholesterol sodium cromoglycate liposomes containing no preservative, demonstrated slight treatment related effects, limited to slight conjunctival redness (Draize grade 1) which was noted in the treated eye of all animals at some point during the dosing period. In all cases this conjunctival redness cleared in less than 24 hours.

C

The test article of the DMPC/cholesterol sodium cromoglycate liposomes containing preservatives BAK and PMA, demonstrated slight treatment related effects, limited to slight conjunctival redness (Draize grade 1) which was noted in the treated eye of three of the four test animals at some point during the dosing period. In all cases this conjunctival redness cleared in less then 24 hours.

Other formulations of liposomal sodium cromoglycate are tabulated at Table 9.

**TABLE 1** SUMMARY OF OCULAR BIODISTRIBUTION DATA FOR CROMOLYN FREE DRUG STUDY

| TISSUE | EYE Rx OR NORx | TIME POINT (HR) | TISSUE CONCENTRATION(ng Cromolyn/g or ml Tissue) EXAMPLE 1 | | | | EXAMPLE 2 | | | | EXAMPLE 3 | | | | WEIGHTED MEAN | SD OF WEIGHTED MEAN |
|---|---|---|---|---|---|---|---|---|---|---|---|---|---|---|---|---|
| | | | MEAN | SD | SEM | N | MEAN | SD | SEM | N | MEAN | SD | SEM | N | | |
| CONJUNCTIVA | Rx | 2 | 3501 | 2951 | 933 | 10 | 3316 | 1790 | 566 | 10 | 2332 | 1640 | 583 | 8 | 2887 | 1122 |
| CONJUNCTIVA | Rx | 4 | 742 | 374 | 125 | 9 | 683 | 318 | 100 | 10 | 772 | 570 | 202 | 8 | 716 | 224 |
| CONJUNCTIVA | Rx | 8 | 687 | 607 | 192 | 10 | 578 | 345 | 109 | 10 | 614 | 560 | 150 | 14 | 607 | 264 |
| NICTITATING MEMBRANE | Rx | 2 | 2136 | 1661 | 525 | 10 | 2438 | 2119 | 670 | 10 | 2618 | 3185 | 1126 | 8 | 2319 | 1216 |
| NICTITATING MEMBRANE | Rx | 4 | 480 | 253 | 84 | 9 | 484 | 387 | 122 | 10 | 576 | 532 | 188 | 8 | 494 | 197 |
| NICTITATING MEMBRANE | Rx | 8 | 348 | 430 | 136 | 10 | 350 | 260 | 82 | 10 | 462 | 347 | 93 | 14 | 383 | 187 |
| CORNES | Rx | 2 | 1005 | 1480 | 468 | 10 | 478 | 237 | 75 | 10 | 683 | 574 | 203 | 8 | 519 | 217 |
| CORNES | Rx | 4 | 487 | 637 | 212 | 9 | 298 | 225 | 71 | 10 | 381 | 336 | 119 | 8 | 337 | 179 |
| CORNES | Rx | 8 | 160 | 184 | 58 | 10 | 165 | 50 | 16 | 10 | 127 | 160 | 43 | 14 | 162 | 47 |
| AQUEOUS HUMOR | Rx | 2 | 70 | 49 | 16 | 10 | 36 | 13 | 4 | 10 | 129 | 129 | 46 | 8 | 39 | 12 |
| AQUEOUS HUMOR | Rx | 4 | 37 | 24 | 8 | 9 | 29 | 28 | 9 | 10 | 68 | 68 | 24 | 8 | 35 | 18 |
| AQUEOUS HUMOR | Rx | 8 | 16 | 21 | 7 | 10 | 13 | 20 | 6 | 10 | 10 | 14 | 4 | 14 | 13 | 10 |
| IRIS-CILIARY BODY | Rx | 2 | ND | | | 10 | ND | | | 10 | 56 | 27 | 10 | 8 | NA | NA |
| IRIS-CILIARY BODY | Rx | 4 | ND | | | 9 | ND | | | 10 | 29 | 42 | 15 | 8 | NA | NA |
| IRIS-CILIARY BODY | Rx | 8 | ND | | | 10 | ND | | | 10 | ND | | | 14 | ND | |
| AQUEOUS HUMOR | NoRX | 2 | ND | | | 10 | ND | | | 10 | ND | | | 8 | ND | |
| AQUEOUS HUMOR | NoRX | 4 | ND | | | 10 | ND | | | 10 | ND | | | 8 | ND | |
| AQUEOUS HUMOR | NoRX | 8 | ND | | | 10 | ND | | | 10 | ND | | | 14 | ND | |
| PLASMA | | 2 | ND | | | 10 | ND | | | 10 | 1 | 3 | 1 | 8 | NA | NA |
| PLASMA | | 4 | ND | | | 9 | ND | | | 10 | ND | | | 8 | ND | |
| PLASMA | | 8 | ND | | | 10 | ND | | | 10 | ND | | | 14 | ND | |

# *TABLE 2*

## CALCULATION OF TISSUE CONCENTRATIONS.

VARIABLE AND CONSTANTS

A = dpm IN TISSUE SAMPLE
B = AVERAGE dpm IN SPIKED TISSUE SAMPLES
C = AVERAGE dpm IN SPIKE
D = AVERAGE dpm IN DOSE
E = MASS(g) OR VOLUME(ml) tissue
1mg = 1 x $10^6$ng=DOSE OF CROMOLYN

CALCULATIONS

FRACTION RADIOACTIVITY RECOVERED = F = B/C
CORRECTED RADIOACTIVITY (dpm) = G = A/F
% DOSE/g OR ml TISSUE = H = 100G/DE
ng CROMOLYN/g OR ml TISSUE* = (1 x $10^6$)H/100

* ASSUMING ALL RADIOACTIVITY IS IN PARENT COMPOUND

TABLE 3

| COMPOSITION OF CHS-BASED CROMOLYN LIPOSOME TESTED IN BIODISTRIBUTION SCREEN. | |
|---|---|
| COMPONENT | CONCENTRATION%(w/v) |
| DISODIUM CROMOGLYCATE | 2.0 |
| SORBIC ACID | 0.05 |
| DISODIUM EDTA | 0.013 |
| SODIUM PHOSPHATE, DIBASIC | 0.71 |
| SODIUM HYDROXIDE | 12.3mEq/100ml(app) |
| CHOLESTERYL ACID SUCCINATE | 10.0 |
| BUTYLATED HYDROXYTOLUENE | 0.001 2.4 |
| STERILE WATER FOR INJECTION, USP | qs |
| pH ≈ 6.5-7.0<br>OSMOLALITY ≈ 240 mmol/kg | |

TABLE 4

| FORMULA OF CHS-BASED CROMOLYN LIPOSOMES WITH SORBIC ACID AND METHYLPARABEN TESTED IN BIODISTRIBUTION SCREEN. | |
|---|---|
| COMPOSITION | |
| COMPONENT | CONCENTRATION%(w/v) |
| DISODIUM CROMOGLYCATE | 2.0 |
| SORBIC ACID | 0.2 |
| METHYLPARABEN | 0.1 |
| DISODIUM EDTA | 0.013 |
| SODIUM PHOSPHATE, DIBASIC | 0.71 |
| SODIUM HYDROXIDE | 12.3mEq/100ml(app) |
| CHOLESTERYL ACID SUCCINATE | 10.0 |
| BUTYLATED HYDROXYTOLUENE | 0.001 |
| STERILE WATER FOR INJECTION, USP | qs |
| pH ≈ 6.5-7.0<br>OSMOLALITY ≈ 240 mmol/kg | |

## *TABLE 10*

GRADING OF OCULAR LESIONS

CORNEA

OPACITY: DEGREE OF DENSITY (AREA MOST DENSE TAKEN FOR READING)

NO ULCERATION OR OPACITY .......... 0

SCATTERED OR DIFFUSE AREAS OF OPACITY (OTHER THAN SLIGHT DULLING OF NORMAL LUSTER), DETAILS OF IRIS CLEARLY VISIBLE .......... 1

EASILY DISCERNIBLE TRANSLUCENT AREA, DETAILS OF IRIS SLIGHTLY OBSCURED .......... 2

NACREOUS AREA, NO DETAILS OF IRIS VISIBLE, SIZE OF PUPIL BARELY DISCERNIBLE .......... 3

OPAOUE CORNEA, IRIS NOT DISCERNIBLE THROUGH OPACITY .......... 4

IRIS

NORMAL .......... 0

MARKEDLY DEEPENED RUGAE, CONGESTION, SWELLING, MODERATE CIRCUMCORNEAL HYPEREMIA OR INJECTION, ANY OF THESE OR COMBINATION THEREOF, IRIS STILL REACTING TO LIGHT (SLUGGISH REACTION IS POSITIVE) .......... 1

NO REACTION TO LIGHT, HEMORRHAGE, GROSS DESTRUCTION (ANY OR ALL OF THESE) .......... 2

CONJUNCTIVA

REDNESS: (REFERS TO PALPEBRAL AND BULBAR CONJUNCTIVAE)

BLOOD VESSELS NORMAL .......... 0

SOME BLOOD VESSELS DEFINITELY HYPEREMIC (INJECTED) .......... 1

DIFFUSE CRIMSON COLOR, INDIVIDUAL VESSELS NOT EASILY DISCERNIBLE .......... 2

DIFFUSE, BEEFY RED .......... 3

CHEMOSIS: LIDS AND/OR NICTITATING MEMBRANE

NO SWELLING .......... 0

ANY SWELLING ABOVE NORMAL (INCLUDES NICTITATING MEMBRANE) .......... 1

OBVIOUS SWELLING WITH PARTIAL EVERSION OF LIDS .......... 2

SWELLING WITH LIDS ABOUT HALF CLOSED .......... 3

SWELLING WITH LIDS MORE THAN HALF-CLOSED .......... 4

## TABLE 10 (CONT.)

DISCHARGE

| | |
|---|---|
| NO DISCHARGE | 0 |
| ANY AMOUNT DIFFERENT FROM NORMAL (DOES NOT INCLUDE SMALL AMOUNTS OBSERVED IN INNER CANTHUS OF NORMAL ANIMALS) | 1 |
| DISCHARGE WITH MOISTENING OF THE LIDS AND HAIRS JUST ADJACENT TO LIDS | 2 |
| DISCHARGE WITH MOISTENING OF THE LIDS AND HAIRS, AND CONSDERABLE AREA AROUND THE EYE | 3 |

TEST SUBSTANCE CLASSIFICATION

| | |
|---|---|
| CATEGORY I | CORROSIVE (IRREVERSIBLE DESTRUCTION OF OCULAR TISSUE) OR IRRITATION PERSISTING MORE THAN 4 DAYS |
| CATEGORY II | CORNEAL INVOLVEMENT AND/OR IRRITATION CLEARING IN 3-4 DAYS |
| CATEGORY III | CORNEAL INVOLVEMENT AND/OR IRRITATION CLEARING IN 2 DAYS OR LESS |
| CATEGORY IV | MINIMAL EFFECTS CLEARING IN LESS THAN 24 HOURS |

## TABLE 20

COMPOSITION OF CHS-BASED CROMOLYN LIPOSOMES SUBMITTED FOR ANTIMICROBIAL EFFECTIVENESS TESTING.

| LIPIDS | | | | PRESERVATIVES | | | | ENHANCER | BUFFER SALTS | | TITRANT | AME |
|---|---|---|---|---|---|---|---|---|---|---|---|---|
| LIPID 1 | (LIPID 1) %(W/Y) | LIPID 2 | (LIPID 2) &(W/V) | PRES 1 | (PRES 1) %(W/V) | PRES 2 | (PRES 2) %(W/Y) | DISODIUM EDTA %(W/Y) | DIBASIC SODIUM PHOSPHATE %(W/Y) | MONOBASIC SODIUM PHOSPHATE %(W/Y) | NaOH (mEqPER 100ml | PASS Y or N |
| CHS | 10 | - | - | BAK | 0.01 | - | - | 0.013 | 0.71 | - | 12.8 | N |
| CHS | 10 | - | - | BAK | 0.01 | PhenAic | 0.4 | 0.013 | 0.71 | - | 12.8 | Y |
| CHS | 10 | - | - | SA | 0.05 | - | - | 0.013 | 0.71 | - | 12.8 | N |
| CHS | 10 | - | - | SA | 0.1 | - | - | 0.013 | 0.71 | - | 12.8 | N |
| CHS | 2.5 | - | - | SA | 0.2 | - | - | 0.013 | 0.71 | - | 12.8 | N |
| CHS | 5 | - | - | SA | 0.2 | - | - | 0.013 | 0.71 | - | 12.8 | N |
| CHS | 10 | - | - | SA | 0.2 | - | - | 0.013 | 0.71 | - | 12.8 | N |
| CHS | 10 | - | - | SA | 0.2 | - | - | 0.013 | 0.71 | - | 12.8 | N |
| CHS | 10 | - | - | SA | 0.2 | MPbn | 0.1 | 0.013 | 0.71 | - | 12.8 | N |

## Claims

**Claims for the following Contracting States : AT, BE, CH, LI, DE, DK, FR, GB, IT, LU, NL, SE**

1. A liposome composition comprising cromolyn and a lipid, wherein the lipid is neutral pH cholesterol hydrogen succinate.

2. The liposome composition of claim 1 additionally containing disodium EDTA and phosphate buffer to a pH around about pH 7.0.

3. The liposome composition of claim 2 additionally containing benzalkonium chloride and phenylethyl alcohol.

4. A liposome composition comprising cromolyn, a phosphate buffer having a pH of about 7.0 and a lipid consisting essentially of a neutral saturated phospholipid and a neutral pH cholesterol hydrogen succinate.

5. The liposome composition of claim 4 additionally comprising disodium EDTA.

6. The liposome composition of claim 4 wherein the phosphate buffer comprises sodium phosphate monobasic and sodium phosphate dibasic.

7. The liposome composition of claim 5 wherein the phosphate buffer comprises sodium phosphate monobasic and sodium phosphate dibasic.

8. The liposome composition of claim 4 wherein the ratio of cromolyn to lipid ranges from about 0.005 to about 20.0.

9. The liposome composition of claim 8 wherein the ratio of cromolyn to lipid ranges from about 0.071 to about 0.667.

10. The liposome composition of claim 9 wherein the ratio of cromolyn to lipid is about 0.2.

11. The liposome composition of claim 7 wherein the saturated lipid is dimyristoylphosphatidylcholine (DMPC), dipalmitoylphosphatidylcholine (DPPC), or hydrogenated soy phosphatidylcholine (HSPC).

12. The liposome composition of claim 6 wherein the saturated lipid is dimyristoylphosphatidylcholine (DMPC), dipalmitoylphosphatidylcholine (DPPC), or hydrogenated soy phosphatidylcholine (HSPC).

13. The liposome composition of claim 11 additionally comprising a preservative.

14. The liposome composition of claim 13 wherein the preservative is phenylmercuric acetate.

15. The liposome composition of claim 13 wherein the preservative is benzalkonium chloride and chlorbutanol.

16. The liposome composition of claim 11 wherein the saturated lipid is dimyristoylphosphatidylcholine additionally comprising phenylmercuric acetate and benzalkonium chloride.

17. The liposome composition of claim 15 wherein the saturated lipid is dimyristoylphosphatidylcholine or hydrogenated soy phosphatidylcholine, additionally comprising methylparaben and propylparaben.

18. The liposome composition of claim 5 wherein the saturated lipid is dimyristoylphosphatidylcholine (DMPC), additionally comprising phenylmercuric acetate, and benzalkonium chloride or phenylethyl alcohol.

19. The liposome composition of claim 5 wherein the saturated phospholipid is dimyristoylphosphatidylcholine (DMPC), additionally comprising benzalkonium chloride and thimersol.

20. A pharmaceutical composition comprising the liposome composition of claim 4 and a pharmaceutically acceptable carrier or diluent.

21. The liposome composition of claim 12 wherein the saturated lipid is hydrogenated soy phosphatidylcholine.

22. The liposome composition of claim 15 wherein the saturated lipid is hydrogenated soy phosphatidylcholine.

**Claims for the following Contracting States : ES, GR**

1. A method for preparing a liposome composition comprising admixing an aqueous phase wherein cromolyn is dissolved and an organic phase wherein a lipid is dissolved in an organic solvent and removing the organic solvent, the lipid comprising neutral pH cholesterol hydrogen succinate.

2. The method of claim 1 wherein the aqueous phase additionally contains disodium EDTA and phosphate buffer to a pH around about pH 7.0.

3. The method of claim 2 wherein the aqueous phase additionally contains benzalkonium chloride and phenylethyl alcohol.

4. A method according to claim 1, wherein the aqueous phase comprises a phosphate buffer having a pH of about 7.0 and the lipid consists essentially of a neutral saturated phospholipid and a neutral pH cholesterol hydrogen succinate.

5. The method of claim 4 wherein the aqueous phase additionally comprises disodium EDTA.

6. The method of claim 4 wherein the phosphate buffer comprises sodium phosphate monobasic and sodium phosphate dibasic.

7. The method of claim 5 wherein the phosphate buffer comprises sodium phosphate monobasic and sodium phosphate dibasic.

8. The method of claim 4 wherein the ratio of cromolyn to lipid ranges from about 0.005 to about 20.0.

9. The method of claim 8 wherein the ratio of cromolyn to lipid ranges from about 0.071 to about 0.667.

10. The method of claim 9 wherein the ratio of cromolyn to lipid is about 0.2.

11. The method of claim 7 wherein the saturated lipid is dimyristoylphosphatidylcholine (DMPC), dipalmitoylphosphatidylcholine (DPPC), or hydrogenated soy phosphatidylcholine(HSPC).

12. The method of claim 6 wherein the saturated lipid is dimyristoylphosphatidylcholine (DMPC), dipalmitoylphosphatidylcholine (DPPC), or hydrogenated soy phosphatidylcholine (HSPC).

13. The method of claim 11 wherein the aqueous phase additionally comprises a preservtive.

14. The method of claim 13 wherein the preservative is phenylmercuric acetate.

15. The method of claim 13 wherein the preservative is benzalkonium chloride and chlorbutanol.

16. The method of claim 11 wherein the saturated lipid is dimyristoylphosphatidylcholine additionally comprising phenylmercuric acetate and benzalkonium chloride.

17. The method of claim 15 wherein the saturated lipid is dimyristoylphosphatidylcholine or hydrogenated soy phosphatidylcholine, additionally comprising methylparaben and propylparaben.

18. The method of claim 5 wherein the saturated lipid is dimyristoylphosphatidylcholine (DMPC), additionally comprising phenylmercuric acetate, and benzalkonium chloride or phenylethyl alcohol.

19. The method of claim 5 wherein the saturated phospholipid is dimyristoylphosphatidylcholine (DMPC), additionally comprising benzalkonium chloride and thimersol.

20. A method for preparing a pharmaceutical composition comprising admixing the liposome composition of claim 4 and a pharmaceutically acceptable carrier or diluent.

21. The method of claim 12 wherein the saturated lipid is hydrogenated soy phosphatidylcholine.

22. The method of claim 15 wherein the saturated lipid is hydrogenated soy phosphatidylcholine.

**Patentansprüche**

**Patentansprüche für folgende Vertragsstaaten : AT, BE, CH, LI, DE, DK, FR, GB, IT, LU, NL, SE**

1. Liposom-Zusammensetzung, die Cromolyn und ein Lipid enthält, wobei das Lipid ein pH-neutrales Cholesterinhydrogensuccinat ist.

2. Liposom-Zusammensetzung nach Anspruch 1, die zusätzlich Dinatrium-EDTA und einen Phosphatpuffer mit einem pH-Wert von etwa 7,0 enthält.

3. Liposom-Zusammensetzung nach Anspruch 2, die zusätzlich Benzalkoniumchlorid und Phenylethylalkohol enthält.

4. Liposom-Zusammensetzung, die Cromolyn, einen Phosphat-Puffer mit einem pH-Wert von etwa 7,0 und ein Lipid enthält, das im wesentlichen besteht aus einem neutralen gesättigten Phospholipid und einem pH-neutralen Cholesterinhydrogensuccinat.

5. Liposom-Zusammensetzung nach Anspruch 4, die zusätzlich Dinatrium-EDTA enthält.

6. Liposom-Zusammensetzung nach Anspruch 4, worin der Phosphatpuffer monobasisches Natriumphosphat und dibasisches Natriumphosphat umfaßt.

7. Liposom-Zusammensetzung nach Anspruch 5, worin der Phosphatpuffer monobasisches Natriumphosphat und dibasisches Natriumphosphat umfaßt.

8. Liposom-Zusammensetzung nach Anspruch 4, worin das Verhältnis von Cromolyn zu Lipid in dem Bereich von etwa 0,005 bis etwa 20,0 liegt.

9. Liposom-Zusammensetzung nach Anspruch 8, worin das Verhältnis von Cromolyn zu Lipid in dem Bereich von etwa 0,071 bis etwa 0,667 liegt.

10. Liposom-Zusammensetzung nach Anspruch 9, worin das Verhältnis von Cromolyn zu Lipid etwa 0,2 beträgt.

11. Liposom-Zusammensetzung nach Anspruch 7, worin das gesättigte Lipid Dimyristoylphosphatidylcholin (DMPC), Dipalmitoylphosphatidylcholin (DPPC) oder hydriertes Sojaphosphatidylcholin (HSPC) ist.

12. Liposom-Zusammensetzung nach Anspruch 6, worin das gesättigte Lipid Dimyristoylphosphatidylcholin (DMPC), Dipalmitoylphosphatidylcholin (DPPC) oder hydriertes Sojaphosphatidylcholin (HSPC) ist.

13. Liposom-Zusammensetzung nach Anspruch 11, die zusätzlich ein Konservierungsmittel enthält.

14. Liposom-Zusammensetzung nach Anspruch 13, worin das Konservierungsmittel Phenylquecksilber(II)-acetat ist.

**15.** Liposom-Zusammensetzung nach Anspruch 13, worin das Konservierungsmittel Benzalkoniumchlorid und Chlorbutanol ist.

**16.** Liposom-Zusammensetzung nach Anspruch 11, worin das gesättigte Lipid Dimyristoylphosphatidylcholin ist, die zusätzlich Phenylquecksilber(II)acetat und Benzalkoniumchlorid enthält.

**17.** Liposom-Zusammensetzung nach Anspruch 15, worin das gesättigte Lipid Dimyristoylphosphatidylcholin oder hydriertes Sojaphosphatidylcholin ist, die zusätzlich Methylparaben und Propylparaben enthält.

**18.** Liposom-Zusammensetzung nach Anspruch 5, worin das gesättigte Lipid Dimyristoylphosphatidylcholin (DMPC) ist, die zusätzlich Phenylquecksilber(II)acetat und Benzalkoniumchlorid oder Phenylethylalkohol enthält.

**19.** Siposom-Zusammensetzung nach Anspruch 5, worin das gesättigte Phospholipid Dimyristoylphosphatidylcholin (DMPC) ist, die zusätzlich Benzalkoniumchlorid und Thimersol enthält.

**20.** Pharmazeutische Zusammensetzung, welche die Liposom-Zusammensetzung nach Anspruch 4 und einen pharmazeutisch akzeptablen Träger oder Verdünnungsmittel enthält.

**21.** Liposom-Zusammensetzung nach Anspruch 12, worin das gesättigte Lipid hydriertes Sojaphosphatidylcholin ist.

**22.** Liposom-Zusammensetzung nach Anspruch 15, worin das gesättigte Lipid hydriertes Sojaphosphatidylcholin ist.

**Patentansprüche für folgende Vertragsstaaten : ES, GR**

**1.** Verfahren zur Herstellung einer Liposom-Zusammensetzung, das umfaßt das Vermischen einer wäßrigen Phase, in der Cromolyn gelöst ist, mit einer organischen Phase, in der ein Lipid in einem organischen Lösungsmittel gelöst ist, und das Entfernen des organischen Lösungsmittels, wobei das Lipid pH-neutrales Cholesterinhydrogensuccinat umfaßt.

**2.** Verfahren nach Anspruch 1, worin die wäßrige Phase zusätzlich Dinatrium-EDTA und einen Phosphatpuffer mit einem pH-Wert von etwa 7,0 enthält.

**3.** Verfahren nach Anspruch 2, worin die wäßrige Phase zusätzlich Benzalkoniumchlorid und Phenylethylalkohol enthält.

**4.** Verfahren nach Anspruch 1, worin die wäßrige Phase einen Phosphat-Puffer mit einem pH-Wert von etwa 7,0 enthält und das Lipid im wesentlichen besteht aus einem neutralen gesättigten Phospholipid und einem pH-neutralen Cholesterinhydrogensuccinat.

**5.** Verfahren nach Anspruch 4, worin die wäßrige Phase zusätzlich Dinatrium-EDTA enthält.

**6.** Verfahren nach Anspruch 4, worin der Phosphatpuffer monobasisches Natriumphosphat und dibasisches Natriumphosphat umfaßt.

**7.** Verfahren nach Anspruch 5, worin der Phosphatpuffer monobasisches Natriumphosphat und dibasisches Natriumphosphat umfaßt.

**8.** Verfahren nach Anspruch 4, worin das Verhältnis von Cromolyn zu Lipid in dem Bereich von etwa 0,005 bis etwa 20,0 liegt.

**9.** Verfahren nach Anspruch 8, worin das Verhältnis von Cromolyn zu Lipid in dem Bereich von etwa 0,071 bis etwa 0,667 liegt.

**10.** Verfahren nach Anspruch 9, worin das Verhältnis von Cromolyn zu Lipid etwa 0,2 beträgt.

11. Verfahren nach Anspruch 7, worin das gesättigte Lipid Dimyristoylphosphatidylcholin (DMPC), Dipalmitoylphosphatidylcholin (DPPC) oder hydriertes Sojaphosphatidylcholin (HSPC) ist.

12. Verfahren nach Anspruch 6, worin das gesättigte Lipid Dimyristoylphosphatidylcholin (DMPC), Dipalmitoylphosphatidylcholin (DPPC) oder hydriertes Sojaphosphatidylcholin (HSPC) ist.

13. Verfahren nach Anspruch 11, worin die wäßrige Phase zusätzlich ein Konservierungsmittel enthält.

14. Verfahren nach Anspruch 13, worin das Konservierungsmittel Phenylquecksilber(II)acetat ist.

15. Verfahren nach Anspruch 13, worin das Konservierungsmittel Benzalkoniumchlorid und Chlorbutanol ist.

16. Verfahren nach Anspruch 11, worin das gesättigte Lipid Dimyristoylphosphatidylcholin ist und zusätzlich Phenylquecksilber(II)acetat und Benzalkoniumchlorid enthält.

17. Verfahren nach Anspruch 15, worin das gesättigte Lipid Dimyristoylphosphatidylcholin oder hydriertes Sojaphosphatidylcholin ist und zusätzlich Methylparaben und Propylparaben enthält.

18. Verfahren nach Anspruch 5, worin das gesättigte Lipid Dimyristoylphosphatidylcholin (DMPC) ist und zusätzlich Phenylquecksilber(II)acetat und Benzalkoniumchlorid oder Phenylethylalkohol enthält.

19. Verfahren nach Anspruch 5, worin das gesättigte Phospholipid Dimyristoylphosphatidylcholin (DMPC) ist und zusätzlich Benzalkoniumchlorid und Thimersol enthält.

20. Verfahren zur Herstellung einer pharmazeutischen Zusammensetzung, das umfaßt das Vermischen der Liposom-Zusammensetzung nach Anspruch 4 mit einem pharmazeutisch akzeptablen Träger oder Verdünnungsmittel.

21. Verfahren nach Anspruch 12, worin das gesättigte Lipid hydriertes Sojaphosphatidylcholin ist.

22. Verfahren nach Anspruch 15, worin das gesättigte Lipid hydriertes Sojaphosphatidylcholin ist.

**Revendications**
**Revendications pour les Etats contractants suivants : AT, BE, CH, LI, DE, DK, FR, GB, IT, LU, NL, SE**

1. Composition de liposome comprenant du cromolyne , et un lipide, dans laquelle le lipide est de l'hydrogéno-succinate de cholestérol à pH neutre.

2. Composition de liposome selon la revendication 1, contenant en outre de l'EDTA disodique et un tampon phosphate à un pH d'environ pH 7,0.

3. Composition de liposome selon la revendication 2, contenant en outre du chlorure de benzalkonium et de l'alcool phényléthyliqrne.

4. Composition de liposome comprenant du cromolyne, un tampon phosphate ayant un pH d'environ 7,0 et un lipide consistant essentiellement en un phospholipide saturé neutre et un hydrogénosuccinate de cholestérol à pH neutre.

5. Composition de liposome selon la revendication 4, comprenant en outre de l'EDTA disodique.

6. Composition de liposome selon la revendication 4, dans laquelle le tampon phosphate comprend du phosphate monosodique et du phosphate disodique.

7. Composition de liposome selon la revendication 5, dans laquelle le tampon phosphate comprend du phosphate monosodique et du phosphate disodique.

8. Composition de liposome selon la revendication 4, dans laquelle le rapport du cromolyne au lipide se situe entre environ 0,005 et environ 20,0.

9. Composition de liposome selon la revendication 8, dans laquelle le rapport du cromolyne au lipide se situe entre environ 0,071 et environ 0,667.

10. Composition de liposome selon la revendication 9, dans laquelle le rapport du cromolyne au lipide est d'environ 0,2.

11. Composition de liposome selon la revendication 7, dans laquelle le lipide saturé est de la dimyristoylphosphatidylcholine (DMPC), de la dipalmitoylphosphatidylcholine (DPPC) ou de la phosphatidylcholine de soja hydrogénée (HSPC).

12. Composition de liposome selon la revendication 6, dans laquelle le lipide saturé est de la dimyristoylphosphatidylcholine (DMPC), de la dipalmitoylphosphatidylcholine (DPPC) ou de la phosphatidylcholine de soja hydrogénée (HSPC).

13. Composition de liposome selon la revendication 11, comprenant en outre un conservateur.

14. Composition de liposome selon la revendication 13, dans laquelle le conservateur est de l'acétate phénylmercurique.

15. Composition de liposome selon la revendication 13, dans laquelle le conservateur est du chlorure de benzalkonium et du chlorobutanol.

16. Composition de liposome selon la revendication 11, dans laquelle le lipide saturé est de la dimyristoylphosphatidylcholine comprenant en outre de l'acétate phénylmercurique et du chlorure de benzalkonium.

17. Composition de liposome selon la revendication 11, dans laquelle le lipide saturé est de la dimyristoylphosphatidylcholine ou de la phosphatidylcholine de soja hydrogénée comprenant en outre du p-hydroxybenzoate de méthyle et du p-hydroxybenzoate de propyle.

18. Composition de liposome selon la revendication 5, dans laquelle le lipide saturé est de la dimyristoylphosphatidylcholine (DMPC), comprenant en outre de l'acétate phénylmercurique et du chlorure de benzalkonium ou de l'alcool phényléthylique.

19. Composition de liposome selon la revendication 5, dans laquelle le phospholipide saturé est de la dimyristoylphosphatidylcholine (DMPC), comprenant en outre du chlorure de benzalkonium et du thimersol.

20. Composition pharmaceutique comprenant la composition de liposome selon la revendication 4 et un support ou diluant pharmmaceutiquement acceptable.

21. Composition de liposome selon la revendication 12, dans laquelle le lipide saturé est de la phosphatidylcholine de soja hydrogénée.

22. Composition de liposome selon la revendication 15, dans laquelle le lipide saturé est de la phosphatidylcholine de soja hydrogénée.

**Revendications pour les Etats contractants suivants : ES, GR**

1. Procédé pour la préparation d'une composition de liposome, comprenant le mélange d'une phase aqueuse dans laquelle du cromolyne, est dissous et d'une phase organique dans laquelle un lipide est dissous dans un solvant organique et l'élimination du solvant organique, le lipide comprenant de l'hydrogénosuccinate de cholestérol à pH neutre.

2. Procédé selon la revendication 1, dans lequel la phase aqueuse contient en outre de l'EDTA disodique et un tampon phosphate à un pH d'environ pH 7,0.

3. Procédé selon la revendication 2, dans lequel la phase aqueuse contient en outre du chlorure de benzalkonium et de l'alcool phényléthylique.

4. Procédé selon la revendication 1, dans lequel la phase aqueuse comprend un tampon phosphate ayant un pH d'environ 7,0 et le lipide consiste essentiellement en un phospholipide saturé neutre et un hydrogénosuccinate de cholestérol à pH neutre.

5. Procédé selon la revendication 4, dans lequel la phase aqueuse comprend en outre de l'EDTA disodique.

6. Procédé selon la revendication 4, dans lequel le tampon phosphate comprend du phosphate monosodique et du phosphate disodique.

7. Procédé selon la revendication 5, dans lequel le tampon phosphate comprend du phosphate monosodique et du phosphate disodique.

8. Procédé selon la revendication 4, dans lequel le rapport du cromolyne au lipide se situe entre environ 0,005 et environ 20,0.

9. Procédé selon la revendication 8, dans lequel le rapport du cromolyne au lipide se situe entre environ 0,071 et environ 0,667.

10. Procédé selon la revendication 9, dans lequel le rapport du cromolyne au lipide est d'environ 0,2.

11. Procédé selon la revendication 7, dans lequel le lipide saturé est de la dimyristoylphosphatidylcholine (DMPC), de la dipalmitoylphosphatidylcholine (DPPC) ou de la phosphatidylcholine de soja hydrogénée (HSPC).

12. Procédé selon la revendication 6, dans lequel le lipide saturé est de la dimyristoylphosphatidylcholine (DMPC), de la dipalmitoylphosphatidylcholine (DPPC) ou de la phosphatidylcholine de soja hydrogénée (HSPC).

13. Procédé selon la revendication 11, dans lequel la phase aqueuse comprend en outre un conservateur.

14. Procédé selon la revendication 13, dans lequel le conservateur est de l'acétate phénylmercurique.

15. Procédé selon la revendication 13, dans lequel le conservateur est du chlorure de benzalkonium et du chlorobutanol.

16. Procédé selon la revendication 11, dans lequel le lipide saturé est de la dimyristoylphosphatidylcholine comprenant en outre de l'acétate phénylmercurique et du chlorure de benzalkonium.

17. Procédé selon la revendication 11, dans lequel le lipide saturé est de la dimyristoylphosphatidylcholine ou de la phosphatidylcholine de soja hydrogénée comprenant en outre du p-hydroxybenzoate de méthyle et du p-hydroxybenzoate de propyle.

18. Procédé selon la revendication 5, dans lequel le lipide saturé est de la dimyristoylphosphatidylcholine (DMPC), comprenant en outre de l'acétate phénylmercurique et du chlorure de benzalkonium ou de l'alcool phényléthylique.

19. Procédé selon la revendication 5, dans lequel le phospholipide saturé est de la dimyristoylphosphatidylcholine (DMPC), comprenant en outre du chlorure de benzalkonium et du thimersol.

20. Procédé pour la préparation d'une composition pharmaceutique comprenant le mélange de la composition de liposome selon la revendication 4 et d'un support ou diluant pharmmaceutiquement acceptable.

21. Procédé selon la revendication 12, dans lequel le lipide saturé est de la phosphatidylcholine de soja hydrogénée.

**22.** Procédé selon la revendication 15, dans lequel le lipide saturé est de la phosphatidylcholine de soja hydrogénée.

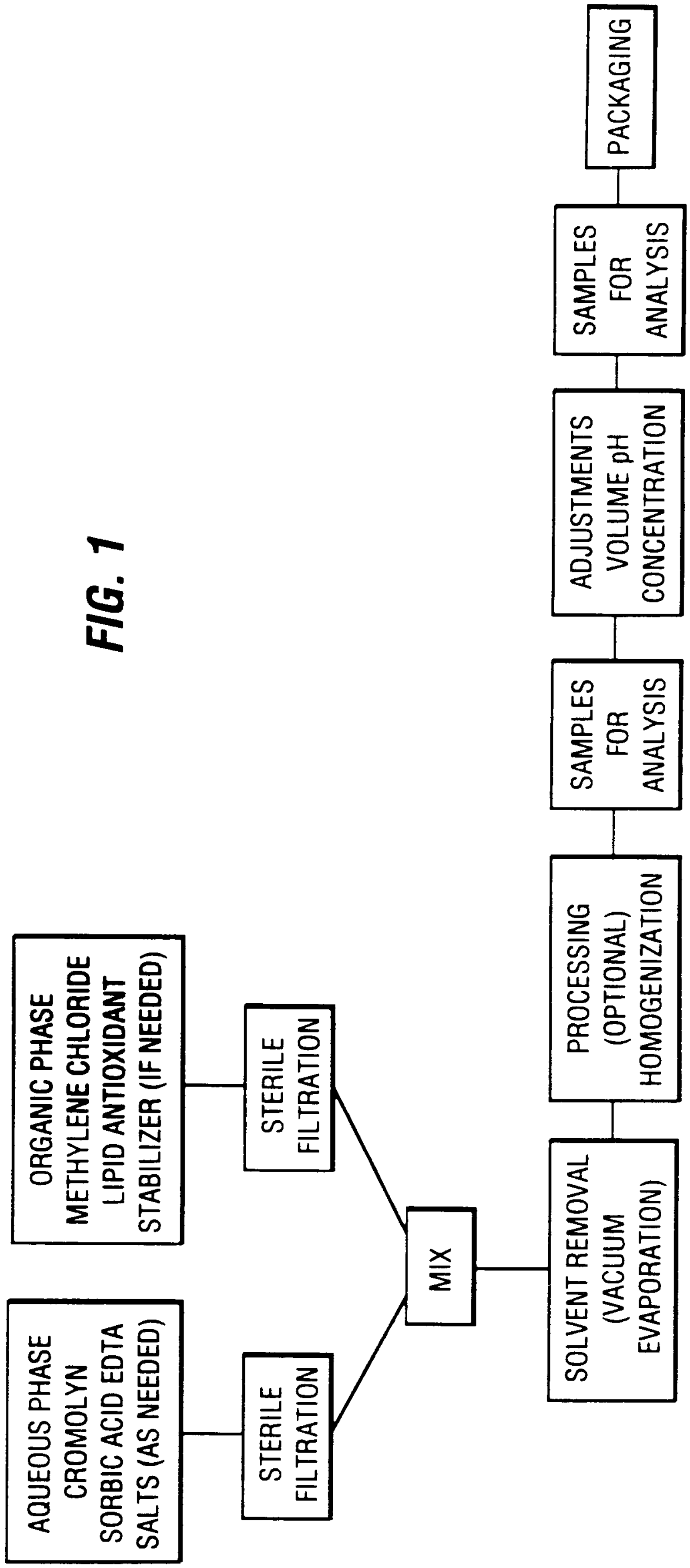
AQUEOUS PHASE
CROMOLYN
SORBIC ACID EDTA
SALTS (AS NEEDED)
ORGANIC PHASE
METHYLENE CHLORIDE
LIPID ANTIOXIDANT
STABILIZER (IF NEEDED)
STERILE FILTRATION
STERILE FILTRATION
MIX
SOLVENT REMOVAL (VACUUM EVAPORATION)
PROCESSING (OPTIONAL) HOMOGENIZATION
SAMPLES FOR ANALYSIS
ADJUSTMENTS VOLUME pH CONCENTRATION
SAMPLES FOR ANALYSIS
PACKAGING

*FIG. 1*

FIG. 2

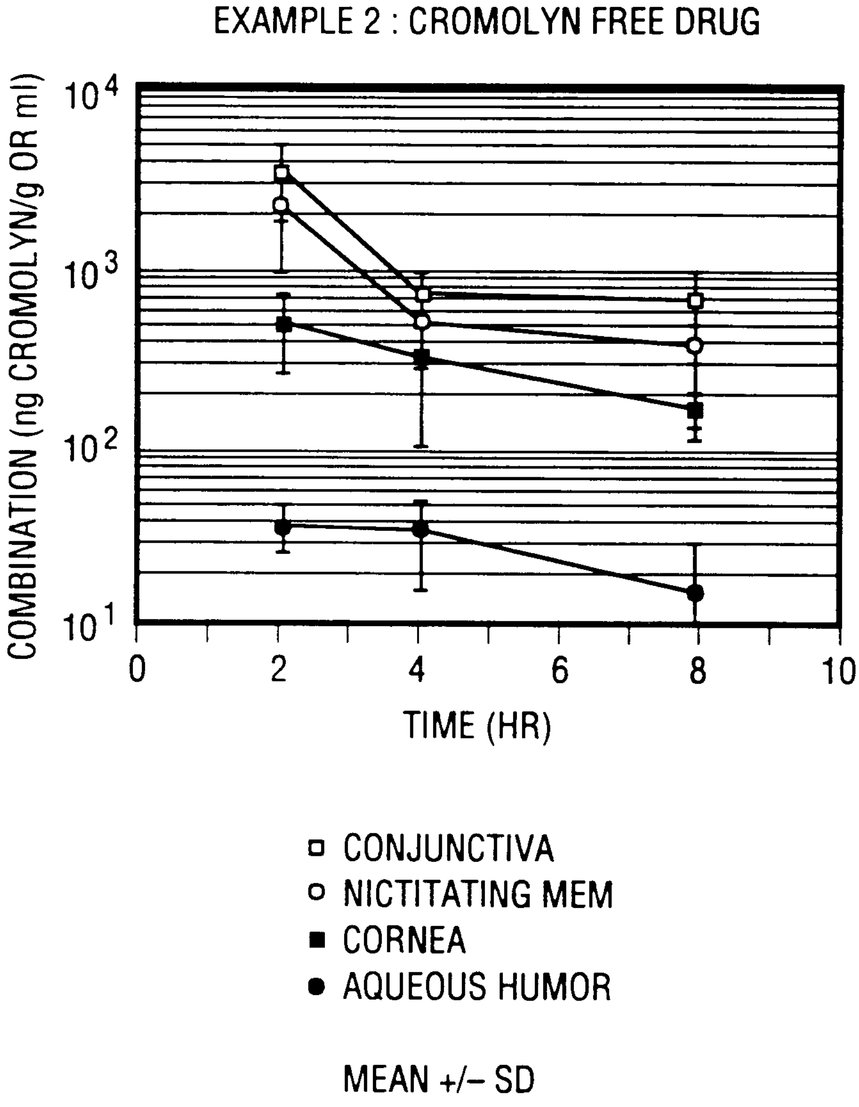
EXAMPLE 2 : CROMOLYN FREE DRUG
COMBINATION (ng CROMOLYN/g OR ml)
$10^4$
$10^3$
$10^2$
$10^1$
0
2
4
6
8
10
TIME (HR)
CONJUNCTIVA
NICTITATING MEM
CORNEA
AQUEOUS HUMOR
MEAN +/- SD